## Europäisches Patentamt
## European Patent Office
## Office européen des brevets

(19)

(11) Numéro de publication: **0 097 571**
**B1**

(12) **FASCICULE DE BREVET EUROPEEN**

(45) Date de publication du fascicule du brevet: **04.12.85**

(21) Numéro de dépôt: **83401176.9**

(22) Date de dépôt: **09.06.83**

(51) Int. Cl.⁴: **A 01 N 63/00**, A 01 N 63/04, C 12 N 1/04, C 12 N 11/04, C 05 F 11/08

(54) Application d'inoculum à faible activité de l'eau à la lutte biologique.

(30) Priorité: **18.06.82 FR 8210636**
**01.04.83 FR 8305442**
**01.04.83 FR 8305443**

(43) Date de publication de la demande:
**04.01.84 Bulletin 84/1**

(45) Mention de la délivrance du brevet:
**04.12.85 Bulletin 85/49**

(84) Etats contractants désignés:
**DE FR GB IT**

(73) Titulaire: **RHONE-POULENC AGROCHIMIE, 14-20, rue Pierre Baizet, F-69009 Lyon (FR)**

(72) Inventeur: **Jung, Gèrard, rue des Grands Jardins Leuville/s/orge, F-93310 Montlhery (FR)**
Inventeur: **Mugnier, Jacques, 63, rue du Cherche Midi, F-75006 Paris (FR)**

(74) Mandataire: **Chrétien, François et al, RHONE-POULENC AGROCHIMIE BP 9163, F-69263 Lyon Cedex 09 (FR)**

(56) Documents cités:
**EP - A - 0 017 565**
**EP - A - 0 083 267**
**FR - A - 2 303 080**
**FR - A - 2 394 606**
**FR - A - 2 501 229**
**GB - A - 2 065 128**

**JOURNAL OF APPLIED BACTERIOLOGY, vol. 35, 1972, pages 493-497, London, GB A. KERR: "Biological control of crown gall: seed inoculation"**
**JOURNAL OF ECONOMIC ENTOMOLOGY, vol. 59, no. 3, juin 1966, pages 620-622, Baltimore, md., US E.S. RAUN et al.: "Encapsulation as a technique for formulating microbial and chemical insecticides"**

(56) Documents cités: (suite)
**The Chemistry of Silica, John Wiley & Sons Edstelns (1979), Table des material**
**Techniques d'analyse et de Control dans les Industries Agro-alimentaires Vol. 4, pages 1 à 60, 1981, Ed. Techniques et Documentation**

BUNDESDRUCKEREI BERLIN

**0 097 571**

## Description

La présente invention concerne l'utilisation d'inoculum pour la lutte biologique. La présente invention concerne plus particulièrement l'utilisation d'inoculum à faible activité de l'eau.

Elle a trait plus particulièrement à un procédé d'inclusion de micro-organismes du genre Agrobacterium radiobacter dans une matrice de gel de polymère, à des fins agronomiques.

La maladie de la galle du collet provoquée par Agrobacterium tumefaciens cause des pertes très importantes en pépinière et en culture fruitière à travers le monde. De très nombreuses dicotylédones sont concernées mais les pertes les plus graves sont occasionnées aux arbres à noyaux et à pépins et aux plantes ornementales.

Le problème économique est d'autant plus grave dans certaines régions produisant des fruits ou de jeunes plantules qu'il n'existe aucun moyen de lutte chimique réellement efficace contre cette maladie. De plus l'exportation des plants atteints vers certains pays est sévèrement réglementée. On a estimé les pertes occasionnées pour les arbres produisant des fruits à noyau en 1974 à 138 millions de dollars U.S.

Ceci souligne l'importance qu'il faut accorder aux autres mesures, notamment au moyen de lutte biologique existant.

Les travaux de KERR (J. Appl. Bact. 35, 493—497, 1972) ont montré qu'il est possible d'utiliser à titre préventif une souche d'Agrobacterium radiobacter var. radiobacter biotype 2, dénommée souche 84 (ou K 84) pour lutter efficacement contre la galle du collet (KERR, Departement of Plant Pathology, Waite Agricultural Research Institute, University of Adelaide, Gen Osmond, South Australia 5064).

Cette souche est commercialisée soit sous forme de culture liquide (KERR, A. 1975, Waite Agricultural Research Institute, Biennal Report, U. Adelaide, p. 4) soit sous forme de culture adsorbée sur de la tourbe (DYE D. W. 1975, Commercial Hort. 7, 5—7), soit sous forme d'un emballage unitaire constitué par une boîte de Pétri (Phytom. Défenses des Cultures, janvier 1982, p. 43).

Dans tous les cas, le concept général suivi pour préparer ces inoculum d'Agrobacterium radiobacter, consiste à considérer que pour assurer la survie de la bactérie, il faut conserver dans les inoculum une certaine humidité.

La présente invention a aussi trait à la lutte biologique contre les insectes par utilisation de poudres constituées de conidiospores de Beauvaria bassiana ou de Metarhizium anisopliae obtenues par culture en milieu solide (FERRON 1978 Biological control of insects pests by entomongenous fongi. Ann. Rev. Entomol. 23, 409—42). Ces spores de champignons entomopathogènes et plus particulièrement les blastospores obtenues à partir du même champignon mais par une technique de culture en milieu liquide sont extrêmement sensibles à la température et à la dessiccation. Des préparations humides ont été obtenues mais elles sont sensibles aux contaminations et ont une durée de conservation limitée à deux mois pour une température de stockage de +4°C, et une conservation très faible pour une température de +23°C (SIEMASZKO Ann. Zool Ecol. anim 1973, 5 (1), p. 69—79).

Suite aux difficultés de stockage de ces préparations humides, différents moyens de séchage ont été tentés afin d'améliorer la durée de conservation de ces blastospores.

Ainsi dans le brevet français n° 7 235 452 publié sous le n° 2 202 159 est décrit un procédé d'obtention de préparations entomopathogènes où les blastospores sont fixées sur un support inerte à base de silice puis déshydratées en présence d'un mélange protecteur à base de glutamate de sodium, de saccharose et d'huile de paraffine contenant un glycéride oléique polyoxyéthyléné. Mais ces préparations doivent être conservées à +4°C ou être conditionnées sous vide ou sous azote.

La présente invention a également trait à la lutte biologique contre un champignon Ceratocystis ulmi responsable de la maladie de l'orme appelée graphiose. En effet, depuis 1918, cette maladie a détruit des millions d'ormes. Ce champignon revêt au cours de son cyle plusieurs formes (mycelium, spores issues du bourgeonnement du mycelium, conidiospores, ascospores) qui toutes sont infectieuses pour l'orme.

On a essayé de lutter contre le principal agent vecteur de cette maladie, c'est-à-dire contre les coléoptères à l'aide d'insecticides chimiques ou biologiques. Ce traitement permet d'éviter la propagation de la maladie mais ne résoud en rien le problème consistant à traiter les ormes malades.

Il est aussi connu d'après le brevet US 4 342 746 de traiter cette maladie par voie biologique par des bactéries capables de se développer dans la sève de l'orme et de produire une substance fortement inhibitrice vis-à-vis de Ceratocystis ulmi. Cette méthode de traitement uniquement biologique a pour avantage d'éviter de nuire à l'environnement en utilisant comme microorganisme le Pseudomonas syringae qui n'est pas toxique ni pour l'homme, ni pour les végétaux.

Afin de développer efficacement cette technique, il est nécessaire de pouvoir disposer de quantités importantes de Pseudomonas syringae.

Mais il est toujours difficile de stocker des organismes vivants sans avoir des pertes importantes. Le stockage de tout microorganisme en milieu liquide est possible mais il doit être réalisé à une température maximale de 4°C et pendant des périodes relativement courtes en évitant toute contamination étrangère ce qui pose de nombreux problèmes techniques et de lourdes contraintes technologiques.

En effet, les divers inoculum décrits dans l'art antérieur, et utilisés en lutte biologique, contiennent,

2

outre le microorganisme, une teneur en eau importante et des éléments nutritifs issus de la culture de la bactérie.

C'est pour cela que ces inoculum demeurent fragiles vis-à-vis des contaminants et résistent mal, même pendant une courte durée, au-delà d'une quarantaine de degrés. Il est nécessaire de les conserver à basse température et leur durée de vie est limitée.

L'objectif de la présente invention est l'application à la lutte biologique de divers inoculum à longue viabilité et à résistance à la température améliorée, caractérisée en ce qu'on inclut le microorganisme dans un gel de polymère et que l'on abaisse l'activité de l'eau en-dessous de 0,3 et de préférence en-dessous de 0,1 et qu'on la maintient à cette valeur.

Les divers inoculum appliqués en lutte biologique peuvent contenir tout microorganisme se présentant sous une forme unicellulaire ou sous forme sporulée. Ainsi peuvent être inclus les bactéries, les champignons choisis en fonction du but à atteindre, c'est-à-dire de la maladie à traiter ou de l'action recherchée. Peuvent notamment être employés des inoculum de bactéries telles que Agrobacterium radiobacter dans la lutte biologique contre la galle du collet, Pseudomonas syringae dans la lutte biologique contre la graphiose de l'orme, des inoculum de champignons entomophatogènes tels que Beauvaria bassiana, Metarhizium anisopliae, Sorosporella uvella, Beauvaria tenella utilisés comme insecticide biologique.

Le moyen pour préparer ces inoculum tel que décrit dans la demande de brevet européen EP-A-83 267 consiste à inclure les microorganismes dans un polymère au moins partiellement réticulé. La préparation de ces inoculum se déroule en 3 étapes:

— on forme au cours de la première étape une solution du polymère et une suspension du microorganisme à inclure;
— on réticule au cours de la deuxième étape au moins partiellement le polymère de manière à inclure le microorganisme dans le gel de polymère;
— on abaisse au cours de la troisième étape l'activité de l'eau;
   Le concept inventif de cette demande de brevet EP-A-83 267 consiste à modifier la disponibilité de l'eau de façon à bloquer son pouvoir calorique et de solvation. Cette disponibilité de l'eau exprimée en terme d'activité de l'eau permet lorsqu'elle atteint des valeurs extrêmement faibles de soustraire les microorganismes aux actions néfastes des solutés présents dans le milieu de culture, des contaminants prêts à se multiplier dans le milieu et au pouvoir de transmission calorique de l'eau.

Cette activité de l'eau minimum au-delà de laquelle les microorganismes seront préservées est fonction des constituants du milieu de culture, du polymère dans lequel sont insérés les microorganismes. Il existe une valeur critique de l'activité de l'eau au-delà de laquelle l'ensemble de ces facteurs présente une influence réduite. Cette valeur de l'activité de l'eau est de 0,3 et de préférence 0,1.

Les valeurs de l'activité de l'eau sont déterminées par la méthode dite des solutions aqueuses saturées (MULTON, 1981, Techniques d'analyse et de contrôle dans les industries agroalimentaires, APRIA) ou par leur mesure directe à l'aide d'un hygromètre (Hygroscop) »ROTRONIC BT«, J. RICHARD et PEKLY. 116 quai de Bezons 95102 ARGENTEUIL.

Les microorganismes pouvant être mis en œuvre selon la présente invention sont choisis notamment parmi les espèces suivantes: Beauvaria bassiana, Metarhizium anisopliae, Sorosporella uvella, Beauvaria tenella, Pseudomonas syringae, Agrobacterium radiobacter.

Le milieu de culture de ces microorganismes renferme au moins une source hydrocarbonée, des sels minéraux, une source azotée et éventuellement des facteurs de croissance.

La source hydrocarbonée est choisie parmi les sucres tels que le glycérol, le sorbitol, le saccharose, le glucose, le fructose; les alcools; les di ou polyosides et les polysaccharides.

La source azotée peut être d'origine minérale telle que nitrate d'ammonium, de potassium ou d'origine organique telle que notamment lysine, arginine, glycine, glutamate, extrait soluble de maïs, extrait de levure.

La concentration de la source hydrocarbonée dans le milieu de culture varie de 10 à 30 g/l, celle de la source azotée varie de 1 à 20 g/l.

L'inclusion des microorganismes dans le polymère selon l'invention peut être réalisée à partir de la culture liquide directement ou à partir d'un concentré obtenu après centrifugation.

On fait subir audit polymère un traitement de réticulation au moins partiel. Par traitement de réticulation au moins partiel, on entend un traitement susceptible de modifier la structure du polymère tel que traitement thermique, traitement par un sel métallique ou par un autre polymère.

Le polymère dans lequel sont inclus les microorganismes doit pour répondre aux projets de normes antipollution être biodégradable. Aussi, les polymères à base de polysaccharides sont utilisés de préférence aux autres polymères. Ils présentent l'avantage d'être rapidement biodégradables, de libérer ainsi facilement les microorganismes, ils influent fortement sur le devenir de la bactérie ou du champignon après inoculation car:

— la taille des particules de polysaccharide permet la meilleure dissémination sur les semences ou le végétal à protéger et permet d'appliquer des quantités d'inoculum minimales;

3

— la poudre de polysaccharide se réhumidifie rapidement;
— le polysaccharide se lie facilement aux particules de sols, aux semences, aux parties de la plante, aux insectes.

Le polymère est avantageusement du groupe des polysaccharides bactérien ou végétal.

Les polysaccharides d'origine bactérienne sont obtenus par fermentation d'un hydrate de carbone par un micro-organisme notamment du genre Xanthomonas ou Arthrobacter ou par des champignons appartenant par exemple au genre Sclerotium.

Les polysaccharides d'origine végétale peuvent avoir des provenances diverses telles que notamment algues (alginates, carraghénanes) exsudats de plantes (gomme karaya, adragante) et de graines (guar, caroube).

La réticulation peut se faire par exemple pour le cas des alginates par des sels métalliques tels que de calcium.

Selon un mode de mise en œuvre particulier de l'invention, on prépare un milieu de culture que l'on ensemence avec la souche de microorganisme. Après quelques jours d'incubation, la culture totale ou les spores ou bactéries obtenues après centrifugation de la culture sont mélangés à un polymère; après réticulation le gel obtenu est déshydraté.

La concentration en polymères dans la suspension à sécher se situe entre 1 et 2%.

On peut ajouter à la suspension avant séchage des additifs tels que les constituants de la source hydrocarbonée précédemment cités ou tout autre produit inerte. L'ensemble microorganisme, gel, sel minéral, source hydrocarbonée et source azotée constitue entre 2 et 10% en poids de la suspension à sécher.

L'étape de deshydratation consiste à diminuer l'activité de l'eau en-dessous de sa valeur critique c'est-à-dire en-dessous de 0,3 et de préférence en dessous de 0,1.

L'étape de deshydratation peut se faire de diverses manières en une ou plusieurs étapes. Selon l'invention, un simple contact à l'air dans les conditions habituelles de température et d'humidité relative n'est pas suffisant.

Selon un premier mode de mise en œuvre de l'étape de déshydratation, on fait appel à des techniques qui permettent d'obtenir une dessiccation plus poussée telles que:

— flux d'air chaud et sec, évidemment dans les conditions compatibles avec la survie du micro-organisme.
— séchage à l'air complété par une deshydratation de la poudre obtenue dans une enceinte hermétiquement close contenant une solution saturée en un composé présentant l'activité de l'eau requise, évoluant vers l'équilibre avec l'inoculum.

Selon un deuxième mode de mise en œuvre de l'étape de déshydratation, on peut faire appel à une technique de séchage par atomisation, qui, de manière inattendue conduit à des inoculum dans lesquels la survie du micro-organisme est préservée alors même que les températures des gaz de traitement sont largement supérieures à celles que le micro-organisme peut subir.

Bien évidemment, on peut combiner plusieurs de ces techniques.

Ainsi du point de vue économique, il est préférable d'adopter un système de séchage bi-étagé comme celui utilisé pour la déshydratation du lait. Ce système se compose d'un séchage par atomisation qui lorsqu'il est effectué avec une température convenable conduit à une activité de l'eau de l'ordre de 0,2 à 0,4 puis un séchage par lit fluidisé jusqu'à notamment une activité de l'eau inférieure à 0,1. L'utilisation d'un système bi-étagé permet d'augmenter le débit de la substance à sécher, par conséquent l'écart de température entre la sortie et l'entrée dans l'atomiseur.

Le lit fluidisé utilisé pour terminer le séchage et atteindre une activité de l'eau inférieure à 0,1 consomme 2 à 4 fois moins d'énergie pour l'élimination de la dernière fraction d'eau que l'atomisation. Globalement l'économie d'énergie réalisée par rapport à un atomiseur simple est de l'ordre de 20%.

Pour maintenir l'activité de l'eau à une valeur optimale, on fait appel à un mode d'emballage étanche limitant les transferts d'eau entre l'atmosphère environnante et l'atmosphère interne des inoculum lors du stockage.

Les inoculum se présentent sous forme de particules, à structure souple, facile à mettre en œuvre, on peut utiliser avantageusement pour leur application la pulvérisation, la remise en suspension dans de l'eau ou dans une solution, l'épandage tel quel sur les sols ou sur les végétaux.

Par ailleurs, dans certains cas, par exemple pour les inoculum à base d'Agrobacterium ou à base de champignons entomophathogènes, on peut apporter l'inoculum sur un support tel que à base de silice.

Dans le cas où l'on fait appel à une méthode impliquant un absorbant tel que la silice, cet absorbant peut en plus remplir une fonction de support et charge facilitant la mise en œuvre et la manipulation de l'inoculum. Le support peut alors avantageusement être constitué d'une silice précipitée de surface BET comprise entre 50 et 600 $m^2/g$.

Dans le cas particulier où l'on introduit une charge minérale présentant des qualités absorbantes telle que par exemple la silice la valeur limite de l'activité de l'eau permettant une conservation des microorganismes est déplacée vers des valeurs plus élevées.

4

Ces inoculum sont utilisés, suivant le microorganisme qu'ils contiennent, comme insecticides, comme fongicides, comme bactéricides, ils sont notamment actifs contre la galle du collet, la graphiose de l'orme.

Mais la présente invention sera plus aisément comprise à l'aide des exemples suivants, donnés à titre indicatif mais nullement limitatif.

## Exemple 1

### 1. Culture d'Agrobacterium radiobacter

La culture est développée pendant 2 jours dans un milieu YEM (A) tel que défini par VINCENT dans: »A manual for practical study of root nodule bacteria« IBP (Handbook 215 Blackwell Scientific Publication Oxfort, page 169) de composition suivante:

| | |
|---|---|
| mannitol | 10 g/l |
| extrait de levure Difco | 1 |
| $K_2HPO_4$ | 0,5 |
| NaCl | 0,2 |
| $MgSO_4$ 7 $H_2O$ | 0,2 |
| $FeCl_3$ | 0,004 |
| eau q.s.p. | 1000 |

La culture est aussi développée dans un milieu B tel que défini par Anand et Heverlein. Dans »American Journal of Botany« 64 (2) p. 153—158, 1977), de composition suivante:

| | |
|---|---|
| saccharose | 10 g/l |
| nutrient Broth Difco | 8 |
| extrait de levure | 1 |

Le PH des milieux est ajusté à 6,8.
On ensemence les milieux avec la souche K 84 (Environmental Protection Agency — USA 38 087-2).

### 2. Préparation d'un inoculum polymère xanthane-farine de graines de caroube

On prépare deux solutions (les valeurs sont données pour la préparation de 300 g d'inoculum).
a) Solution de gomme xanthane: polysaccharide de type anionique de PM $> 2 \cdot 10^6$ résultant de la fermentation d'hydrates de carbone par un micro-organisme de genre Xanthomonas que l'on désignera par produit 1.
On amène 100 ml d'eau distillée à 70—80°C, on ajoute 1,5 g du produit 1, on maintient cette température 20 à 30 minutes, sous agitation puis on le ramène entre 40 et 50°C.
b) Solution de farine de graine de caroube = polysaccharide constitué d'unités — D — manno pyranosyl (liaisons 1—4) une sur quatre ou cinq étant substituée en $C_6$ par un — D — galacto-pyranosyl PM = 3,1 $10^5$ que l'on désignera par la suite par produit 2.
On procède de la même façon en remplaçant le produit 1 par 1,5 g de farine de graine de caroube.
Lorsque les deux solutions sont à 40—45°C, on ajoute sous agitation, à chacune d'elle, 50 ml de suspension d'Agrobacterium radiobacter. On verse alors, en agitant vigoureusement le mélange culture + gomme xanthane dans le mélange culture + farine de graines de caroube. L'obtention d'un gel consistant est instantanée.
On obtient 2 types d'inoculum A et B suivant qu'on introduit la suspension de bactéries provenant du milieu de culture A ou B.

### 3. Préparation d'un inoculum polymère alginate $CaSO_4$

Deux étapes sont nécessaires:

a) Préparation de la suspension d'Agrobacterium radiobacter dans un alginate de viscosité comprise entre 150 $10^{-3}$ et 1000 $10^{-3}$ Pa · s. On dissout dans 80 ml de culture bactérienne provenant du milieu de culture A. 1 g d'alginate. Pour cela, on disperse la poudre en pluie fine sur la culture en l'agitant continuellement jusqu'à solubilisation complète de l'alginate.

b) Gélification
On ajoute à la suspension de spores d'Agrobacterium radiobacter dans l'alginate 20 ml d'une solution de $CaSO_4$ 2 $H_2O$ à 6 g/l. La prise en masse du gel est instantanée. On obtient un inoculum C.

Comme dit précédemment, le séchage est un point important puisque les modes de séchage utilisés dans l'art antérieur pour la survie des microorganismes tels qu'Agrobacterium radiobacter conduisaient à la destruction des bactéries.

Dans les exemples suivants, on règle l'activité de l'eau au moyen de solutions saturées dont les valeurs des activités de l'eau à une température de 25°C sont exposées dans le tableau I.

| Solutes (solutions saturées) | $a_w$ = Activité de l'eau à 25°C |
|---|---|
| Actigel (composé déshydratant) | 0,04 |
| NaOH | 0,07 |
| $KC_2H_3O_2 - 1,5\ H_2O$ | 0,23 |
| $CaCl_2 - 6\ H_2O$ | 0,31 |
| $K_2CO_3 - 2\ H_2O$ | 0,43 |
| $Ca(NO_3)_2\ 4\ H_2O$ | 0,51 |
| $CuCl_2$ | 0,68 |
| NaCl | 0,75 |
| KCl | 0,84 |

On a illustré dans les diverses courbes de survie l'influence d'un certain nombre de paramètres.

Figure 1:
Elle représente les courbes de survie des inoculum A (courbe 1) et B (courbe 2) après 15 Jours de stockage à 28°C, en fonction de l'activité de l'eau.
Ces courbes montrent que la survie d'Agrobacterium radiobacter est maximale lorsque l'activité de l'eau dans l'inoculum est la plus faible, elles montrent en outre, l'importance de la composition du milieu de culture sur la survie de la bactérie.
Les composés du milieu de culture contenus dans l'inoculum B sont plus néfastes pour la survie du microorganisme que ceux de l'inoculum A.

Figure 2:
Elle représente les courbes de survie de l'inoculum A à deux activités de l'eau ($1\ a_w$: 0,03 et $2\ a_w$: 0,11) au cours du stockage à 28°C.
La survie d'Agrobacterium radiobacter est possible dans un biopolymère déshydraté pendant une période d'au moins un an.

Figure 3:
Elle représente les courbes de survie d'Agrobacterium radiobacter de l'inoculum A en fonction de l'activité de l'eau au cours du stockage à 55°C ($1\ a_w = 0,03$; $2\ a_w = 0,07$; $3\ a_w = 0,23$; $4\ a_w = 0,31$ et $5\ a_w = 0,43$). Elle illustre la résistance de la bactérie dans les biopolymères et notamment lors d'un traitement thermique important.

Figure 4:
Elle représente l'évolution de l'activité de l'eau dans l'inoculum A en fonction de la durée de stockage à 38°C et à HR = 90% ensaché dans différents matériaux d'emballage (1. emballage polypropylène (RP Films); 2. emballage métallisé polypropylène (RP Films) et 3. emballage métallisé en polyester-propylène (Clarylène® RP Films).
Le 3ème type d'emballage est totalement étanche à la vapeur d'eau, et la figure montre l'importance de l'étanchéité de l'emballage.
Le tableau II illustre un essai de contrôle biologique par saupoudrage des inoculum A d'Agrobacterium radiobacter, souche 84 au niveau d'une plaie d'inoculation effectuée à la base de la tige des plants de tomate. Les plants ont été préalablement infectés par une souche d'Agrobacterium tumefaciens virulente (C58 pTi C58, Phabagen Collection. Department of molecular cell biology. De Mithof 3 584 CH UTRECHT, The Netherlands).

Cet essai montre que la prévention de la galle du collet causé par une souche virulente est de 100% lorsque l'on applique préventivement des inoculum de bactéries antagonistes inclus dans un biopolymère déshydraté.

Tableau II

Contrôle biologique de la galle du collet de plants de tomate

| Inoculum (Agrobacterium tumefaciens) | Poids des galles du collet (g/plant) | | | | | | | | | | |
|---|---|---|---|---|---|---|---|---|---|---|---|
| C58 pTi C58 (0,2 ml culture liquide/plant) | 4,4 | 3,7 | 2,1 | 4,1 | 2,8 | 4,2 | 4,0 | 3,6 | 5,1 | 3,1 | 2,1 |
| C58 pTi C58 × Agrobacterium radiobacter 84 (culture liquide) 0,2 ml/plant | 0 | 0 | 0 | 0 | 0 | 0 | 0 | 0 | 0 | 0 | 0 |
| Agrobacterium radiobacter 84 Inoculum A 2 mg/plant | 0 | 0 | 0 | 0 | 0 | 0 | 0 | 0 | 0 | 0 | 0 |

Age des plantules: 60 j.
Culture sur milieu vermiculite perlite + NPK et oligoéléments.

Exemple 2

On ensemence le milieu avec un champignon entomopathogène: Beauvaria bassiana souche Bb 169 (INRA).

### 1. Culture de blastospores de Beauvaria bassiana

La culture des blastospores est développée durant 5 jours en milieu à base d'extrait soluble de maïs/saccharose de composition suivante: (CATROUX et al. 1970) Ann. Zoologie et Ecologie animale T2 p. 281 – 294, 1970.

| | |
|---|---|
| extrait soluble de maïs | 20 g/l |
| saccharose | 30 |
| $KNO_3$ | 5 |
| $KH_2PO_4$ | 6,8 |
| $CaCO_3$ | 0,2 |
| $MgSO_4$ 7 $H_2O$ | 0,1 |
| $H_2O$ q.s.p. | 1000 |

### 2. Préparation d'un inoculum polymère xanthane-farine de graines de caroube

On procède comme à l'exemple 1 en remplaçant les 50 ml de suspension d'Agrobacterium radiobacter par 50 ml de suspension de spores de Beauvaria bassiana.

### 3. Préparation d'un inoculum polymère alginate

On procède de la même façon qu'à l'exemple 1 en remplaçant la suspension d'Agrobacterium radiobacter par une suspension de spores de Beauvaria bassiana.

### 4. Stockage des gels ainsi obtenus

On sèche les gels ainsi obtenus à l'air puis on les place dans différentes enceintes contenant une solution saturée telle que définie dans le tableau I.

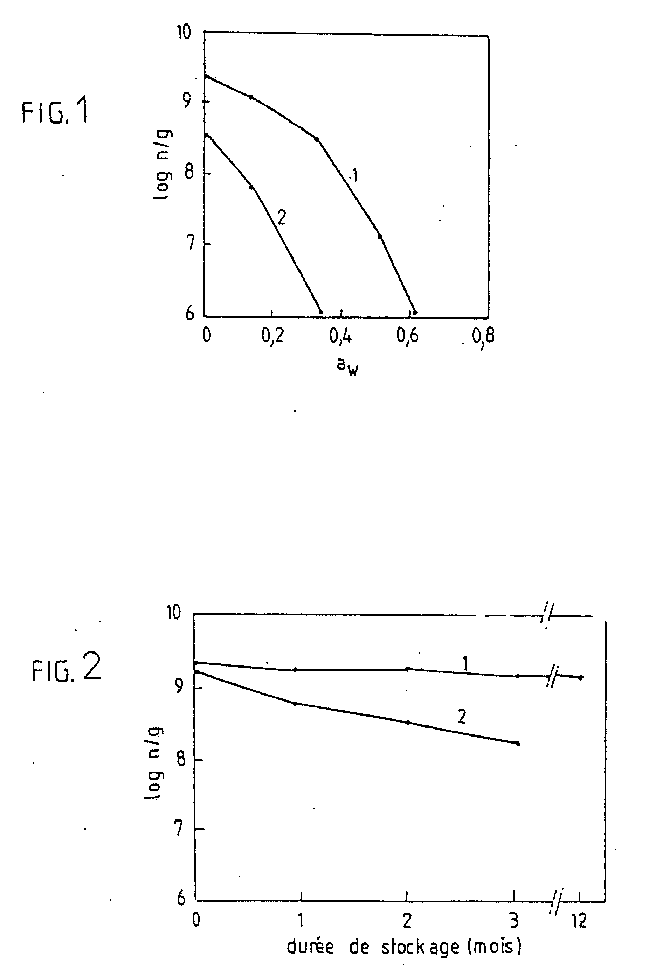

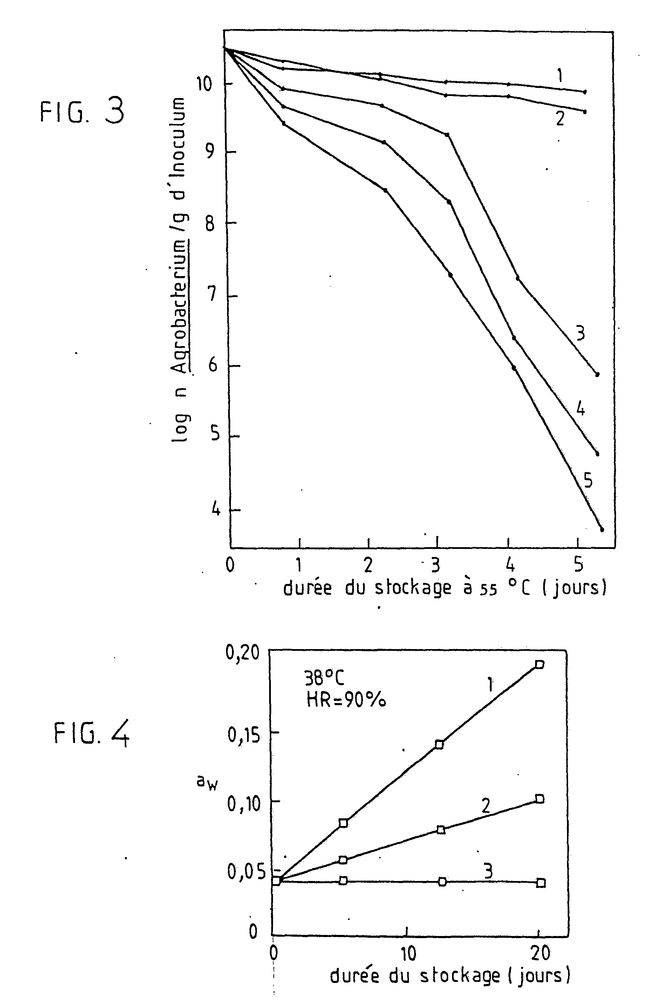

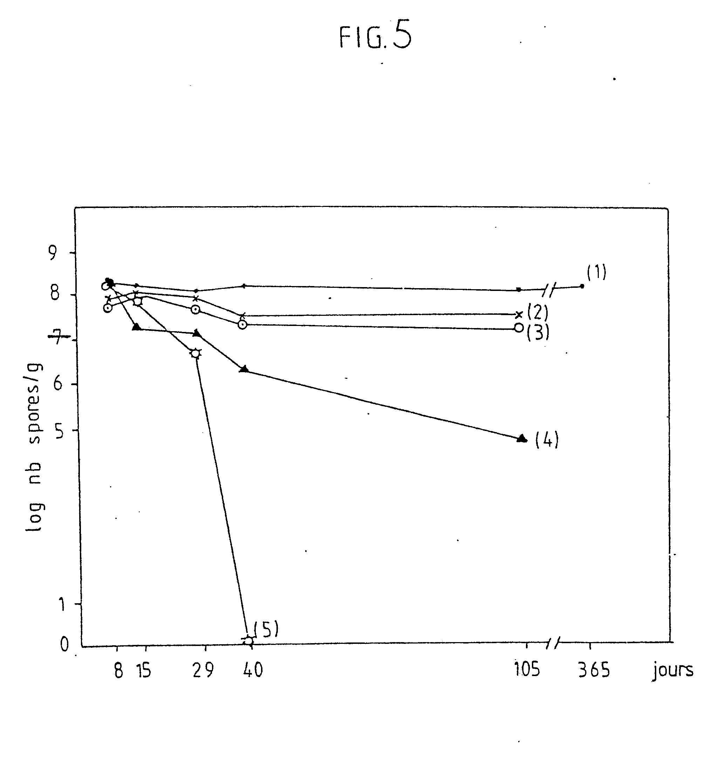

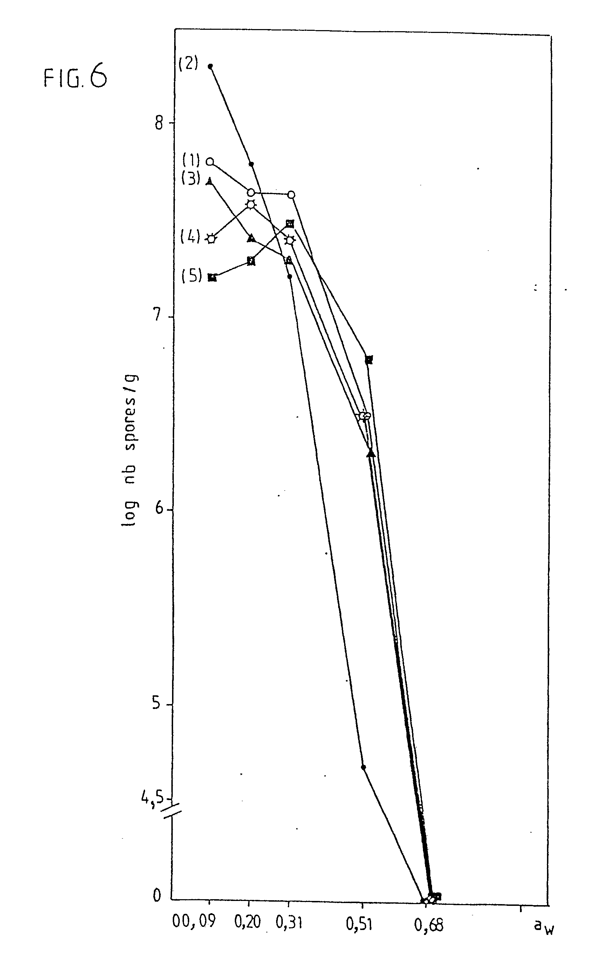

La figure 5 représente l'allure des courbes de survie des blastospores incluses dans un gel gomme xanthane-farine de graines de caroube placées à ces différentes activités de l'eau:

| Courbe | $a_w$ |
| --- | --- |
| 1 | 0,07 |
| 2 | 0,23 |
| 3 | 0,31 |
| 4 | 0,51 |
| 5 | 0,68 |

— à $a_w$ = 0,07 la survie des blastospores est de 100% durant au moins un an de stockage à 28° C.
— à $a_w$ = 0,23 et 0,31 la perte des blastospores est de 90% environ après 100 jours de stockage à 28° C.

La figure 6 représente l'allure des courbes de survie des blastospores incluses dans des gels d'alginate après addition de 10 à 40% du poids du gel de silice naturelle ou synthétique, séchage et stockage 105 jours à 28° C à différentes activités de l'eau.

| Courbe (1) | addition de silice (I) à une culture de Beauvaria bassiana |
| Courbe (2) | spores sans additif |
| Courbe (3) | spores + silice FLO |
| Courbe (4) | spores + silice (I) |
| Courbe (5) | spores + silice (II) |

On a choisi 3 types de silice:

— une silice naturelle (silice Clarcel FLO à base de perlite)
— une silice précipitée — silice (I) — commercialisée sous la marque Zeosil 45 présentant:
   — une surface BET     250 m$^2$/g
   — une prise d'huile     320 cm$^3$/100 g
   — CTAB     170 m$^2$/g
— une silice expérimentale — silice (II) — présentant:
   — une surface BET     200 m$^2$/g
   — une prise d'huile     350 cm$^3$/100 g
   — CTAB     90 m$^2$/g

La surface CTAB (surface externe) par adsorption de bromure de céthyl triméthyl ammonium à pH 9 est déterminée selon la méthode exposée par JAY, JANSEN et G. RAUS dans Rubber Chemistry and Technology 44 (1971) pages 1287 — 1296.

La présence de silice permet une conservation des blastospores de Beauvaria bassiana jusqu'à une activité de l'eau de 0,3 donc augmentée par rapport au gel contenant les spores sans additif.

### Exemple 3

On ensemence le milieu avec la souche de Pseudomonas syringae M 27 fournie par Ortho Chemical Company. Cette souche est déposée à la Northen Utilization Research and Development Division sous le numéro NRRL B 12050 et à l'Institut Pasteur sous le numéro I 142.

**0 097 571**

### 1. Culture de Pseudomonas syringae

La culture est développée durant trois jours sur gélose. Cette culture sert à ensemencer un erlenmeyer de 300 ml contenant un milieu de culture YEM qui correspond au milieu (A) tel que défini précédemment pour la culture d'Agrobacterium radiobacter.

Après trois jours d'incubation à 28° C, on ensemence un erlenmeyer de 2000 ml contenant le milieu YEM précédemment décrit. Après 24 heures, on ensemence un fermenteur de 75 litres contenant le même milieu. Après 24 heures d'incubation, le nombre de germes obtenu est de $3,2 \cdot 10^9$ germes/ml.

### 2. et 3. Préparation d'un inoculum polymère xanthane-farine de graines de caroube et d'un inoculum polymère alginate

On procède de la même façon qu'à l'exemple 1 en remplaçant la suspension d'Agrobacterium radiobacter par une suspension de Pseudomonas syringae.

### 4. Stockage des gels obtenus

On sèche les gels ainsi obtenus à l'air puis on les place dans différentes enceintes contenant une solution saturée telle que définie dans le tableau I.

Avant stockage et après séchage, le logarithme du nombre de germes par gramme est de 9,3.

On place les gels séchés contenant Pseudomonas syringae dans des enceintes correspondant aux activités de l'eau suivantes: 0,04 — 0,07 — 0,23 — 0,31 — 0,51 — 0,75 — 0,84 pendant 90 jours à une température de 28° C.

A la suite de cette période (90 jours) les bactéries sont dénombrées par la méthode des suspensions dilutions bien connue de l'homme de l'art. Les résultats obtenus (tableau III) montrent que Pseudomonas syringae présente une très bonne survie au cours du temps lorsqu'il est stocké à une activité de l'eau ($a_w$) inférieure ou égale à 0,07 et une survie correcte lorsqu'il est stocké à une activité de l'eau inférieure à 0,3.

| $a_w$ | 0,04 | 0,07 | 0,23 | 0,31 | 0,51 | 0,75 | 0,84 |
|---|---|---|---|---|---|---|---|
| log nb g/g | 9,2 | 9,2 | 8,4 | 7,1 | 0 | 0 | 0 |

### 5. Activité de Pseudomonas syringae sur Ceratocystis ulmi

On mesure l'activité de suspensions de Pseudomonas syringae obtenues à partir d'une culture de Pseudomonas syringae incluse dans un gel d'alginate séché et stocké 80 jours à une activité de l'eau de 0,07 puis remise en suspensions à raison de 60 mg de poudre déshydratée pour 9 ml de tampon pH 7, par estimation de l'inhibition de la croissance de Ceratocystis ulmi en boîte de Petri.

Le pourcentage d'inhibition de la culture atteint 90% par rapport à un témoin développé dans les mêmes conditions en absence de Pseudomonas syringae inclus.

L'activité de Pseudomonas syringae sur Ceratocystis ulmi est parfaitement conservée après un stockage de 80 jours à une activité de l'eau de 0,07.

### 6. Influence de la source hydrocarbonée sur la survie de Pseudomonas syringae

Des milieux de culture contenant des sources hydrocarbonées différentes ont été essayés. La suspension de Pseudomonas syringae obtenue a été incluse dans un gel d'alginate qui a été séché puis stocké pendant 122 jours à 28° C à une activité de l'eau de l'ordre de 0,07.

Le nombre de bactéries survivantes a été déterminé par la technique des suspensions dilutions.

Le milieu de culture assurant une survie optimale de Pseudomonas syringae est le milieu YEM contenant comme source hydrocarbonée le mannitol.

7. Comparaison de l'efficacité de diverses souches de Pseudomonas syringae sur Ceratocystis ulmi

Les souches essayées ont été fournies par = Ortho Chevron Chemical company — Institut National de la Recherche Agronomique — Collection Nationale de Bactéries Phytopathogènes (C.N.B.P.).

| Souches de Pseudomonas syringae | Nb. de germes par ml | | Inhibition C. Ulmi % |
|---|---|---|---|
| Chevron M 27 | 2,5 | $10^9$ | 90 |
| INRA 62311 | 3,0 | $10^9$ | 0 |
| INRA 62224 | 2,2 | $10^9$ | 0 |
| INRA 64611 | 1,0 | $10^9$ | 60 |
| INRA 5911 | 3,4 | $10^9$ | 0 |
| CNBP 1585 | 1,9 | $10^9$ | 80 |

**Revendications**

1. Application d'inoculum à la lutte biologique, caractérisée en ce qu'on inclut le microorganisme dans un gel de polymère et que l'on abaisse l'activité de l'eau en-dessous de 0,3 et de préférence en-dessous de 0,1 et qu'on la maintient à cette valeur.

2. Application selon la revendication 1, à la lutte biologique contre la galle du collet, caractérisée en ce qu'on inclut Agrobacterium radiobacter dans un gel de polymère et que l'on abaisse l'activité de l'eau en-dessous de 0,3 et de préférence en-dessous de 0,1 et qu'on la maintient à cette valeur.

3. Application selon la revendication 1, contre les insectes, caractérisée en ce qu'on inclut un champignon entomopathogène dans un gel de polymère et que l'on abaisse l'activité de l'eau en-dessous de 0,3 et de préférence en-dessous de 0,1 et qu'on la maintient à cette valeur.

4. Application selon la revendication 3, caractérisée en ce que le champignon entomopathogène est choisi parmi : Beauvaria bassiana, Metarhizium anisopliae, Sorosporella uvella, Beauvaria tenella.

5. Application selon la revendication 1, à la lutte biologique contre la graphiose de l'orme, caractérisée en ce qu'on inclut Pseudomonas syringae dans un gel de polymère et que l'on abaisse l'activité de l'eau en-dessous de 0,3 et de préférence en-dessous de 0,1 et qu'on la maintient à cette valeur.

6. Application selon la revendication 1, caractérisée en ce que le polymère est du groupe des polysaccharides auquel on fait subir une réticulation au moins partielle.

7. Application selon la revendication 1 ou 6, caractérisée en ce que le polymère est réticulé par l'un des modes de traitement suivants : thermique, à l'aide d'un sel métallique au moyen d'un autre polymère.

8. Application selon la revendication 1 ou 6, caractérisée en ce que le polymère est un polymère à base de gomme xanthane et de farine de caroube.

9. Application selon la revendication 1 ou 6, caractérisée en ce que le polymère est un polymère à base d'alginate.

10. Application selon la revendication 1, caractérisée en ce que le milieu de culture de microorganismes contient une source hydrocarbonée choisie parmi les sucres, les alcools, les di ou polyosides et les polysaccharides.

11. Application selon la revendication 1, caractérisée en ce que le milieu de culture des microorganismes contient une source azotée minérale telle que le nitrate d'ammonium, de potassium ou organique telle que la lysine, l'arginine, la glycine et le glutamate, l'extrait soluble de maïs, l'extrait de levure.

12. Application selon la revendication 1, caractérisée en ce que l'activité de l'eau est abaissée par atomisation.

13. Application selon la revendication 1, caractérisée en ce que l'activité de l'eau est abaissée par atomisation et passage sur un lit fluidisé.

14. Application selon les revendications 2, 3 ou 4, caractérisée en ce que l'inoculum est apporté sur un support à base de silice.

15. Application selon la revendication 14, caractérisée en ce que le support est constitué par une silice précipitée de surface BET comprise entre 50 et 600 m²/g.

# 0 097 571

## Patentansprüche

1. Verwendung eines Inokulums zur biologischen Bekämpfung, dadurch gekennzeichnet, daß der Mikroorganismus in ein Polymergel eingeschlossen wird, die Wasseraktivität auf unter 0,3 und vorzugsweise auf unter 0,1 gesenkt und auf diesem Wert gehalten wird.

2. Verwendung nach Anspruch 1 zur biologischen Bekämpfung der Pflanzenwurzelhalsgalle, dadurch gekennzeichnet, daß Agrobacterium radiobacter in ein Polymergel eingeschlossen wird, die Wasseraktivität auf unter 0,3 und vorzugsweise auf unter 0,1 gesenkt und auf diesem Wert gehalten wird.

3. Verwendung nach Anspruch 1 zur Bekämpfung von Insekten, dadurch gekennzeichnet, daß ein entomopathogener Pilz in ein Polymergel eingeschlossen wird, die Wasseraktivität auf unter 0,3 und vorzugsweise auf unter 0,1 gesenkt und auf diesem Wert gehalten wird.

4. Verwendung nach Anspruch 3, dadurch gekennzeichnet, daß der entomopathogene Pilz unter Beauvaria bassiana, Metarhizium anisopliae, Sorosporella uvella und Beauvaria tenella ausgewählt wird.

5. Verwendung nach Anspruch 1 zur biologischen Bekämpfung der Ulmenkrankheit (Graphiose), dadurch gekennzeichnet, daß Pseudomonas syringae in ein Polymergel eingeschlossen wird, die Wasseraktivität auf unter 0,3 und vorzugsweise auf unter 0,1 gesenkt und auf diesem Wert gehalten wird.

6. Verwendung nach Anspruch 1, dadurch gekennzeichnet, daß das Polymer ein Polysaccharid ist, das zumindest teilweise vernetzt worden ist.

7. Verwendung nach Anspruch 1 oder 6, dadurch gekennzeichnet, daß das Polymer thermisch, mit Hilfe eines Metallsalzes oder mit einem anderen Polymer vernetzt worden ist.

8. Verwendung nach Anspruch 1 oder 6, dadurch gekennzeichnet, daß das Polymer auf Xanthangummi- oder Johannisbrotmehl-Basis ist.

9. Verwendung nach Anspruch 1 oder 6, dadurch gekennzeichnet, daß das Polymer auf Alginat-Basis ist.

10. Verwendung nach Anspruch 1, dadurch gekennzeichnet, daß das Kulturmedium für die Mikroorganismen eine Kohlenwasserstoffquelle, wie Zucker, Alkohole, Di- oder Polyoside und/oder Polysaccharide, enthält.

11. Verwendung nach Anspruch 1, dadurch gekennzeichnet, daß das Kulturmedium für die Mikroorganismen eine anorganische Stickstoffquelle, wie Ammonium- oder Kaliumnitrat, oder eine organische Stickstoffquelle, wie Lysin, Arginin, Glycin, Glutamat, einen löslichen Maisextrakt oder einen Hefeextrakt enthält.

12. Verwendung nach Anspruch 1, dadurch gekennzeichnet, daß die Wasseraktivität durch Atomisierung gesenkt wird.

13. Verwendung nach Anspruch 1, dadurch gekennzeichnet, daß die Wasseraktivität durch Atomisierung und Leiten durch ein Wirbelbett gesenkt wird.

14. Verwendung nach Anspruch 2, 3 oder 4, dadurch gekennzeichnet, daß das Inokulum auf einem Siliciumdioxid-Träger eingebracht wird.

15. Verwendung nach Anspruch 14, dadurch gekennzeichnet, daß der Träger aus ausgefälltem Siliciumdioxid mit einer BET-Fläche von 50 bis 600 $m^2/g$ besteht.

## Claims

1. Use of inoculum for biological control characterized in that a micro-organism is included in a polymer gel and the water activity is brought to below 0.3 and preferably below 0.1 and kept at that level.

2. Use as claimed in claim 1 for biological control of crown gall characterized in that Agrobacterium radiobacter is included in a polymer gel and that the water activity is brought to below 0.3 and preferably below 0.1 and kept at that level.

3. Use as claimed in claim 1 for insect control characterized in that an entomopathogenic fungus is included in a polymer gel and that the water activity is brought to below 0.3 and preferably below 0.1 and kept at that level.

4. Use as claimed in claim 3 characterized in that the entomopathogenic fungus is chosen amongst Beauvaria bassiana, Metarhizium anisopliae, Sorosporella uvella or Beauvaria tenella.

5. Use as claimed in claim 1 for biological control of elm graphiosis, characterized in that Pseudomonas syringae is included in a polymer gel and that the water activity is brought to below 0.3 and preferably below 0.1 and kept at that level.

6. Use as claimed in claim 1 characterized in that the polymer is a polysaccharide that has been subjected to at least partial cross-linking.

7. Use as claimed in claim 1 or 6 characterized in that the polymer is cross-linked according to one of the following treatments: thermal with the aid of a metal salt, or by means of a different polymer.

8. Use as claimed in claim 1 or 6 characterized in that the polymer is based on xanthan gum and carob bean flour.

9. Use as claimed in claim 1 or 6 characterized in that the polymer is based on alginate.

10. Use as claimed in claim 1 characterized in that the culture medium used for the micro-organisms contains a hydrocarbon source, chosen amongst sugars, alcohols, di- or poly-osides or polysaccharides.

11

**0 097 571**

11. Use as claimed in claim 1 characterized in that the culture medium used for the micro-organisms contains a inorganic nitrogen source such as ammonium or potassium nitrate, or a organic nitrogen source such as lysine, arginine, glycine, a glutamate, soluble maize extract or yeast extrat.

12. Use as claimed in claim 1 characterized in that the water activity is reduced by atomisation.

13. Use as claimed in claim 1 characterized in that the water activity is reduced by atomisation and passing over a fluidised bed.

14. Use as claimed in any one of claims 2 to 4 characterized in that the inoculum is brought on a silica-based carrier.

15. Use as claimed in claim 14 characterized in that the carrier is constituted by a precipitated silica with a BET surface of from 50 to 600 m$^2$/g.

FIG. 1

FIG. 2

FIG. 3

FIG. 4

# FIG.5

FIG. 6